# EUROPEAN PATENT SPECIFICATION

(11) **EP 2 766 009 B1**
(45) Date of publication and mention of the grant of the patent: **09.10.2019**
(21) Application number: 11873918.4
(22) Date of filing: 11.10.2011
(51) Int. Cl.: A61K 31/185, A61K 31/19, A61K 36/28, A61K 8/30, A61K 8/36, A61K 8/97, A61P 17/00, A61Q 19/00

(54) **METHOD AND COMPOSITIONS FOR TREATING SKIN**
VERFAHREN UND ZUSAMMENSETZUNGEN ZUR HAUTBEHANDLUNG
PROCÉDÉ ET COMPOSITIONS DE TRAITEMENT DE LA PEAU

(43) Date of publication of application: 20.08.2014
(73) Proprietor: ELC Management LLC, New York, NY 10153 (US)
(72) Inventor: PELLE, Edward (Dr.), Valley Stream New York 11580 (US); PERNODET, Nadine A., Huntington Station New York 11746 (US); YAROSH, Daniel B., Merrick New York 11566 (US)
(74) Representative: Dehns
(86) International application number: PCT/US2011/055676
(87) International publication number: WO 2013/055315

(56) References cited:
- WO-A1-2012/000957
- JP-A- 2006 241 034
- US-A1- 2003 223 982
- US-A1- 2004 170 581
- MAFFEI FACINO R ET AL: "Echinacoside and caffeoyl conjugates protect collagen from free radical-induced degradation: A potential use of Echinacea extracts in the prevention of skin photodamage", PLANTA MEDICA, THIEME VERLAG, DE, vol. 61, no. 6, 1 January 1995 (1995-01-01), pages 510-514, XP009118013, ISSN: 0032-0943
- CHIEN-YU WANG ET AL.: 'Genomics and proteomics of immune modulatory effects of a butanol fraction of echinacea purpurea in human dendritic cells.' BMC GENOMICS vol. 9, no. 479, 13 October 2008, pages 1471 - 2164, XP021042217
- MARIA MIJULASOVA ET AL.: 'GENOTOXIC EFFECTS OF THE HYDROXYCINNAMIC ACID DERIVATIVES &#8 211; caffeic, chlorogenic and cichoric acids.' BIOLOGIA BRATISLAVA. vol. 60, no. 3, 2005, pages 275 - 280, XP008173219

## Description

### Technical Field

The invention is in the field of methods for treating skin cells to increase perl gene expression in order to ameliorate the adverse effects of natural aging of skin, or UV radiation.

### Background of the Invention

It is well known that environmental aggressors such as UV radiation, environmental pollution, environmental toxins, physiological stress, and the natural process of aging can be very detrimental to skin. The skin on the face is made up of keratinocytes, fibroblasts, melanocytes, T-cells and so on. Environmental aggressors cause damage to DNA of skin cells and affects the cellular circadian rhythm in general. The body's natural circadian rhythms are synchronized such that during exposure to environmental aggressors-usually during daylight hours - certain genes in the cells are activated to produce proteins that protect the cells against damage.

Genes associated with natural bodily circadian rhythms have been identified and include the clock (Circadian Locomotor Output Cycles Kaput) gene and the perl (Period Homolog 1) gene, both of which encode proteins (CLK and PERI) that regulate circadian rhythms. Clock and perl genes are also present in skin cells. The induction of perl gene expression initiates a program of cellular activity that is associated with biological processes that take place at night (e.g. repair). It is known that skin cells exposed to environmental aggressors will often exhibit decreased, irregular, or asynchronous clock or perl gene expression. This in turn causes disruption of normal circadian rhythm in the exposed skin cells. Over a prolonged period of time disruption of normal cellular circadian rhythm and synchronicity can-+ accelerate the natural aging process of skin which leads to wrinkles, fine lines, skin laxity, uneven pigmentation, age spots, mottling, and the like.

For this reason it is very advantageous to maintain natural cellular circadian rhythm to the greatest extent possible. To date, the only known mechanisms for synchronizing skin cells are to (1) starve the cells for an extended period of time by removing nutrients and energy sources, and then suddenly resupply the nutrients; or (2) treating the skin cells with a peptide having the C.T.F.A. name Tripeptide-32. The practical difficulties in starving skin cells not in culture are obvious. In addition, the ingredient known to have this activity is expensive.

It has been discovered that cichoric acid is an effective stimulator of perl gene expression in skin cells. The ability to increase perl gene expression in skin cells means that skin cells with irregular or decreased synchronicity will become synchronized (e.g. all cells exhibit same level of perl gene expression at the same time). When skin cells are synchronized, treatment of the skin cells with active ingredients that repair cellular damage are the most effective. It is most beneficial to treat skin cells to increase perl gene expression and thereby cause the treated cells to be synchronized so that the active ingredients in skin treatment products may be optimally beneficial to the skin cells. In one most preferred embodiment of the invention the skin cells are treated with a cichoric acid-containing composition as defined in claim 1 to increase perl gene expression and synchronicity at night prior to retiring.

### Summary of the Invention

The invention is directed to a cosmetic method for treating adverse effects of the natural aging process of the skin, the method comprising increasing and/or synchronizing perl gene expression in skin cells having decreased, irregular, or asynchronous perl gene expression due to the natural aging process by treating the skin cells with a composition comprising an effective amount of cichoric acid, and a DNA repair enzyme;
wherein the cichoric acid is in the form of an extract of *Echinacea purpurea*;
wherein the DNA repair enzyme is in the form of *Bifida* Ferment Lysate; and
wherein the composition is in the form of a skin cream, lotion, foundation makeup, lipstick, concealer, blush, serum, eye shadow, cleanser or toner.

The invention is also directed to a composition for use in a method for increasing and/or synchronizing perl gene expression in skin cells having decreased, irregular, or asynchronous per1 gene expression due to exposure to UV radiation comprising treating the skin cells with the composition as defined in claim 5 which comprises cichoric acid in an amount sufficient to increase the perl gene expression of the treated cells.

The method as defined in claim 5 may comprise:
(a) During the day treating the skin cells with a composition containing chemical or physical UV sunscreens;
(b) At night, treating skin cells that may have decreased, irregular, or asynchronous perl gene expression due to UV exposure during day hours, with the composition comprising cichoric acid in an amount sufficient to increase and/or synchronize the perl gene expression of the treated cells.

### Brief Description of the Figures

Figure 1 depicts the perl gene expression of *Echinacea purpurea* extract.
Figure 2 depicts the perl gene expression of cichoric acid.

### Detailed Description

### I. Definitions

All percentages mentioned herein are percentages by weight unless otherwise indicated.

"Environmental pollution" means contaminants typically found in the environment such as smog, cigarette smoke, dust, pollen, motor vehicle exhaust, and the like.

"Environmental toxins" means ingredients found in products that consumers may use in day to day life such as cleaning products containing solvents, chemicals found in contaminated ground water, plastic by products, and the like.

The term "DNA repair enzyme" means an enzyme that is operable to repair DNA base mutagenic damage. Such enzymes are often categorized by the type of DNA damage they repair, for example base excision repair (BER) enzymes, nucleotide excision repair (NER) enzymes; mismatch repair (MMR) enzymes; DNA helicases; DNA polymerases, and so on. For example, DNA lesions such as 8-oxo-7,8-dihydro-2'-deoxyguanosine may be repaired by OGG1 (8-oxoGuanine glycosylase); T-T dimers which may be repaired by NER (nucleotide excision repair) or CPD Photolyase); 6-4 photoproducts (which may be repaired by NER or 6-4 Photolyase); and 06-methyl guanine (which may be repaired by 06-alkyl guanine DNA transferase (AGT)). The DNA repair enzyme in the composition used in the invention is in the form of *Bifida* Ferment lysate.

"Natural aging process" means the natural process of skin aging which includes formation of wrinkles, fine lines, skin laxity, uneven pigmentation, skin mottling, yellowness, and so on.

"per1 gene expression" means the expression of per1 genes in skin cells which may be measured by, among other things, synthesis of proteins transcribed by that gene.

"Physiological stress" means stress conditions to which skin cells may be exposed such as wind burn, itching, chafing, extreme heat or cold.

"skin cells" when used herein means cells that make up skin including but not limited to keratinocyes, melanocytes, fibroblasts, T-cells, and so on.

The term "synchronizing perl gene expression" means that the perl gene expression of the treated skin cells is synchronized.

"UV radiation" means ultraviolet radiation in the UVA and UVB wavelength ranges.

### II. The Method of the Invention

In the method of the invention cichoric acid in the form of an extract from *Echinacea purpurea* is applied to skin cells, preferably those found on the face or body skin in an amount sufficient to increase and/or synchronize the perl gene expression in the treated cells. The cichoric acid is incorporated into a cosmetic composition and that composition is used to treat skin cells, preferably keratinocytes. In such a case, suggested ranges of cichoric acid are from about 0.000001 to about 40%, preferably from about 0.000005 to 35%, more preferably from about 0.00001 to 25%.

The term "cichoric acid" when used herein also includes any isomers thereof that are operable to increase perl gene expression in skin cells.

The compositions used in the invention comprise cichoric acid in the form of an extract from *Echinacea purpurea.* Most preferred is a botanical extract from *Echinacea purpurea* sold by Symrise under the brand name Symfinity™ 1298 which is an extract of *Echinacea purpurea* which is standardized during the extraction process to contain about 3% by weight of the total extract composition of cichoric acid. *Echinacea* extracts from different sources will vary in cichoric acid content, and as such will yield variable results in induction of perl gene expression. For example, we have observed that another component commonly found in extracts of *Echinacea,* specifically caftaric acid, does not increase perl gene expression in skin cells. Moreover, each species of *Echinacea* will differ in content of phenolic and cichoric acids. Ethanolic extract of the roots of *Echinacea purpura* will provide more cichoric acid than ethanolic extracts of *Echineacea angustifolia* or *Echinacea pallida.* The content of active ingredients in any extract is also very dependent on the method of extraction. For example, it is known that in many cases enzymatic browning during the extraction process will reduce the phenolic acid content of the resulting extract.

The cichoric acid containing composition used in the invention is applied to the skin in the form of a skin cream, lotion, foundation makeup, lipstick, concealer, blush, serum, eye shadow, cleanser or toner. The composition may be applied to the skin one or more times per day and in any regimen. The cichoric acid may be found in skin cream or lotion or in one or more of a cleanser or toner. Preferably the cichoric acid-containing composition is applied to the skin at night, prior to retiring, to maximize the skin's natural repair process.

The composition into which the cichoric acid is formulated contains a DNA repair enzyme in the form of *Bifida* Ferment Lysate and may contain other ingredients including but not limited to those set forth herein.

### III. DNA Repair Enzymes

The composition used and for use in the methods of the invention also contains at least one DNA repair enzyme in the form of *Bifida* Ferment Lysate, and optionally other DNA repair enzymes. Suggested ranges are from about 0.00001 to about 35%, preferably from about 0.00005 to about 30%, more preferably from about 0.0001 to about 25% of one or more DNA repair enzymes.

Other DNA repair enzymes as disclosed in U.S. Patent Nos. 5,077,211; 5,190,762; 5,272,079; and 5,296,231 are suitable for use in the compositions and method of the invention. One example of such a DNA repair enzyme may be purchased from AGI/Dermatics under the trade name Roxisomes®, and has the INCI name *Arabidopsis Thaliana* extract. It may be present alone or in admixture with lecithin and water. This DNA repair enzyme is known to be effective in repairing 8-oxo-Guanine base mutation damage.

Another type of DNA repair enzyme that may optionally be used is one that is known to be effective in repairing 06-methyl guanine base mutation damage. It is sold by AGI/Dermatics under the tradename Adasomes®, and has the INCI name Lactobacillus ferment, which may be added to the composition of the invention by itself or in admixture with lecithin and water.

Another type of DNA repair enzyme that may optionally be used is one that is known to be effective in repairing T-T dimers. The enzymes are present in mixtures of biological or botanical materials. Examples of such ingredients are sold by AGI/Dermatics under the tradenames Ultrasomes® or Photosomes®. Ultrasomes® comprises a mixture of *Micrococcus* lysate (an end product of the controlled lysis of various species of micrococcus), lecithin, and water. Photosomes® comprises a mixture of plankton extract (which is the extract of marine biomass which includes one or more of the following organisms: thalassoplankton, green micro-algae, diatoms, greenish-blue and nitrogen-fixing seaweed), water, and lecithin.

The compositions used in the invention comprise a DNA repair enzyme in the form of *Bifida* ferment lysate, which is a lysate from *Bifido* bacteria which contains the metabolic products and cytoplasmic fractions when *Bifido* bacteria are cultured, inactivated and then disintegrated. This material has the INCI name *Bifida* Ferment Lysate.

Other suitable optional DNA repair enzymes include T4 Endonuclease V, which may be produced by the denV gene of the bacteriophage T4. Also suitable are base glycosylases such as uracil- and hypoxanthine-DNA glycosylases; apyrimidinic/apurinic endonucleases; DNA exonucleases, damaged-bases glycosylases (e.g., 3-methyladenine-DNA glycosylase); correndonucleases either alone or in complexes (e.g., E. coli uvrA/uvrB/uvrC endonuclease complex); APEX nuclease, which is a multi-functional DNA repair enzyme often referred to as "APE"; dihydrofolate reductase; terminal transferase; topoisomerase.

Other types of suitable optional DNA repair enzymes may be categorized by the type of repair facilitated and include BER (base excision repair) or BER factor enzymes such as uracil-DNA glycosylase (UNG); single strand selective monofunctional uracil DNA glycosylase (SMUG1); 3,N(4)-ethenocytosine glycosylase (MBD4); thymine DNA-glycosylase (TDG); A/G-specific adenine DNA glycosylase (MUTYH); 8-oxoguanine DNA glycosylase (OGG1); endonuclease III-like (NTHL1); 3-methyladenine DNA glycosidase (MPG); DNA glycosylase/AP lyase (NEIL1 or 2); AP endonuclease (APEX 1 and 2), DNA ligase (LIG3), ligase accessory factor (XRCC1); DNA 5'-kinase/3'-phosphatase (PNKP); ADP-ribosyltransferase (PARP1 or 2).

Another category of optional DNA repair enzymes includes those that are believed to directly reverse methylpurine damage such as 1-meA dioxygenase (ALKBH2 or ALKBH3).

Yet another category of enzymes operable to repair DNA/protein crosslinks includes Tyr-DNA phosphodiesterase (TDP1).

Also suitable as optional ingredients are MMR (mismatch excision repair) DNA repair enzymes such as MutS protein homolog (MSH2); mismatch repair protein (MSH3); mutS homolog 4 (MSH4); MutS homolog 5 (MSH5); or G/T mismatch-binding protein (MSH6); DNA mismatch repair protein (PMS1, PMS2, MLH1, MLH3); Postmeiotic segregation increased 2-like protein (PMS2L3); or postmeiotic segregation increased 2-like 4 pseudogene (PMS2L4).

Also suitable as optional ingredients are DNA repair enzymes known as nucleotide excision repair (NER) enzymes and include those such as Xeroderma pigmentosum group C-complementing protein (XPC); RAD23 (S. cerevisiae) homolog (RAD23B); caltractin isoform (CETN2); RFA Protein 1, 2, of 3 (RPA1, 2, or 3); 3' to 5' DNA helicase (ERCC3); 5' to 3' DNA helicase (ERCC2); basic transcription factor (GTF2H1, GTF2H2, GTF2H3, GTF2H4, GTF2H5); CDK activating kinase (CDK7, CCNH); cyclin G1-interacting protein (MNAT1); DNA excision repair protein ERCC-51; excision repair cross-complementing 1 (ERCC1); DNA ligase 1 (LIG1); ATP-dependent helicase (ERCC6); and the like.

Also suitable as optional ingredients may be DNA repair enzymes in the category that facilitate homologous recombination and include, but are not limited to DNA repair protein RAD51 homolog (RAD51, RAD51L1, RAD51B etc.); DNA repair protein XRCC2; DNA repair protein XRCC3; DNA repair protein RAD52; ATPase (RAD50); 3' exonuclease (MRE11A); and so on.

DNA repair enzymes that are DNA polymerases are also suitable as optional ingredients and include DNA polymerase beta subunit (POLB); DNA polymerase gamma (POLG); DNA polymerase subunit delta (POLD1); DNA polymerase II subunit A (POLE); DNA polymerase delta auxiliary protein (PCNA); DNA polymerase zeta (POLZ); MAD2 homolog ((REV7); DNA polymerase eta (POLH): DNA polymerase kappa (POLK): and the like.

Various types of DNA repair enzymes that are often referred to as "editing and processing nucleases" include 3'-nuclease; 3'-exonuclease; 5'-exonuclease; endonuclease; and the like.

Other examples of DNA repair enzymes include DNA helicases including such as ATP DNA helicase and so on.

The optional DNA repair enzymes may be present as components of botanical extracts, bacterial or yeast lysates, biological materials, and the like. For example, botanical extracts may contain DNA repair enzymes.

The composition used in the invention may contain one or more DNA repair enzymes, provided they contain a DNA repair enzyme in the form of *Bifida* Ferment Lysate. Preferably, the composition contains other ingredients that will provide a cosmetically or pharmaceutically acceptable product.

### IV. Other Ingredients

The composition used in the method of the invention may be in the form of an emulsion, aqueous solution or dispersion, gel, or anhydrous composition. If in the form of an emulsion, it may be a water-in-oil or oil-in-water emulsion. If in the form of an emulsion, the composition may contain from about 1-99%, preferably from about 5-90%, more preferably from about 10-85% water and from about 1-99%, preferably from about 5-90%, more preferably from about 5-75% of oil. If in the form of an aqueous suspension or dispersion, the composition may generally contain from about 1-99.9%, preferably from about 5-95%, more preferably from about 10-90% water, with the remaining ingredients being the active ingredients or other formula ingredients.

### A. Humectants

The composition used in the method of the invention may contain one or more humectants. If present, they may range from about 0.1 to 75%, preferably from about 0.5 to 70%, more preferably from about 0.5 to 40%. Examples of suitable humectants include glycols, sugars, and the like. Suitable glycols are in monomeric or polymeric form and include polyethylene and polypropylene glycols such as PEG 4-10, which are polyethylene glycols having from 4 to 10 repeating ethylene oxide units; as well as C₁₋₆ alkylene glycols such as propylene glycol, butylene glycol, pentylene glycol, and the like. Suitable sugars, some of which are also polyhydric alcohols, are also suitable humectants. Examples of such sugars include glucose, fructose, honey, hydrogenated honey, inositol, maltose, mannitol, maltitol, sorbitol, sucrose, xylitol, xylose, and so on. Also suitable is urea. Preferably, the humectants used in the composition of the invention are C₁₋₆, preferably C₂₋₄ alkylene glycols, most particularly butylene glycol.

### B. Surfactants

It may be desirable for the composition used in the method of the invention to contain one more surfactants, especially if in the emulsion form. However, such surfactants may be used if the compositions are solutions, suspensions, or anhydrous also, and will assist in dispersing ingredients that have polarity, for example pigments. Such surfactants may be silicone or organic based. The surfactants will also aid in the formation of stable emulsions of either the water-in-oil or oil-in-water form. If present, the surfactant may range from about 0.001 to 30%, preferably from about 0.005 to 25%, more preferably from about 0.1 to 20% by weight of the total composition.

### 1. Organic Nonionic Surfactants

The composition used in the method of the invention may comprise one or more nonionic organic surfactants. Suitable nonionic surfactants include alkoxylated alcohols or ethers, formed by the reaction of an alcohol with an alkylene oxide, usually ethylene or propylene oxide. Suitable alcohols include mono-, di-, or polyhydric short chain (C1-6) alcohols; aromatic or aliphatic saturated or unsaturated fatty (C12-40) alcohols, of cholesterol; and so on.

Cholesterol is suitable, or an aromatic or aliphatic saturated or unsaturated fatty alcohol which may have from 6 to 40, preferably from about 10 to 30, more preferably from about 12 to 22 carbon atoms. Examples include oleyl alcohol, cetearyl alcohol, cetyl alcohol, stearyl alcohol, isostearyl alcohol, behenyl alcohol, and the like. Examples of such ingredients include Oleth 2-100; Steareth 2-100; Beheneth 5-30; Ceteareth 2-100; Ceteth 2-100; Choleth 2-100 wherein the number range means the number of repeating ethylene oxide units, e.g. Ceteth 2-100 means Ceteth where the number of repeating ethylene oxide units ranges from 2 to 100. Derivatives of alkoxylated alcohols are also suitable, such as phosphoric acid esters thereof.

Some preferred organic nonionic surfactants include Oleth-3, Oleth-5, Oleth-3 phosphate, Choleth-24; Ceteth-24; and so on.

Also suitable are alkoxylated alcohols formed with mono-, di-, or polyhydric short chain alcohols, for example those having from about 1 to 6 carbon atoms. Examples include glucose, glycerin, or alkylated derivatives thereof. Examples include glycereth 2-100; gluceth 2-100; methyl gluceth 2-100 and so on. More preferred are methyl gluceth-20; glycereth-26 and the like.

Other types of alkoxylated alcohols are suitable surfactants, including ethylene oxide polymers having varying numbers of repeating EO groups, generally referred to as PEG 12 to 200. More preferred are PEG-75, which is may be purchased from Dow Chemical under the trade name Carbowax PEG-3350.

Other suitable nonionic surfactants include alkoxylated sorbitan and alkoxylated sorbitan derivatives. For example, alkoxylation, in particular ethoxylation of sorbitan provides polyalkoxylated sorbitan derivatives. Esterification of polyalkoxylated sorbitan provides sorbitan esters such as the polysorbates. For example, the polyalkyoxylated sorbitan can be esterified with C6-30, preferably C12-22 fatty acids. Examples of such ingredients include Polysorbates 20-85, sorbitan oleate, sorbitan sesquioleate, sorbitan palmitate, sorbitan sesquiisostearate, sorbitan stearate, and so on.

### 2. Silicone or Silane Surfactants

Also suitable are various types of silicone or silane-based surfactants. Examples include organosiloxanes substituted with ethylene oxide or propylene oxide groups such as PEG dimethicones which are dimethicones substituted with polyethylene glycols including those having the INCI names PEG-1 dimethicone; PEG-4 dimethicone; PEG-8 dimethicone; PEG-12 dimethicone; PEG-20 dimethicone; and so on.

Also suitable are silanes substituted with ethoxy groups or propoxy groups or both, such as various types of PEG methyl ether silanes such as bis-PEG-18 methyl ether dimethyl silane; and so on.

Further examples of silicone based surfactants include those having the generic names dimethicone copolyol; cetyl dimethicone copolyol; and so on.

### C. Botanical Extracts

It may be desirable to incorporate one more additional botanical extracts (other than the extract from *Echinacea purpurea* that contains cichoric acid as a component) into the composition. If present suggested ranges are from about 0.0001 to 20%, preferably from about 0.0005 to 15%, more preferably from about 0.001 to 10%. Suitable botanical extracts include extracts from plants (herbs, roots, flowers, fruits, seeds) such as flowers, fruits, vegetables, and so on, including yeast ferment extract, *Padina pavonica* extract, *Thermus thermophilis* ferment extract, *Camelina sativa* seed oil, *Boswellia serrata* extract, olive extract, *Acacia dealbata* extract, *Acer saccharinum* (sugar maple), *Acidopholus, Acorus, Aesculus, Agaricus, Agave, Agrimonia,* algae, aloe, citrus, *Brassica,* cinnamon, orange, apple, blueberry, cranberry, peach, pear, lemon, lime, pea, seaweed, caffeine, green tea, chamomile, willowbark, mulberry, poppy, and those set forth on pages 1646 through 1660 of the CTFA Cosmetic Ingredient Handbook, Eighth Edition, Volume 2. Further specific examples include, but are not limited to, *Glycyrrhiza glabra, Salix nigra, Macrocycstis pyrifera, Pyrus malus, Saxifraga sarmentosa, Vitis vinifera, Morus nigra, Scutellaria baicalensis, Anthemis nobilis, Salvia sclarea, Rosmarinus ojficianalis, Citrus limonum, Panax ginseng, Siegesbeckia orientalis, Fructus mume, Ascophyllum nodosum, Glycine soja* extract, *Beta vulgaris, Haberlea rhodopensis, Polygonum cuspidatum, Citrus aurantium dulcis, Vitis vinifera, Selaginella tamariscina, Humulus lupulus, Citrus reticulata* Peel, *Punica granatum, Asparagopsis, Curcuma longa, Menyanthes trifoliata, Helianthus annuus,* Hordeum vulgare, *Cucumis sativus, Evernia prunastri, Evernia furfuracea, Kola acuminata,* and mixtures thereof.

### D. Biological Materials

Also suitable are various types of biological materials such as those derived from cells, fermented materials, and so on. If present such materials may range from about 0.001 to 30%, preferably from about 0.005 to 25%, more preferably from about 0.01 to 20%. Examples include fragments of cellular RNA or DNA, or probiotic microorganisms. Particularly preferred are RNA fragments.

### E. Oils

In the event the compositions used in the method of the invention are in emulsion form, the composition will comprise an oil phase. Oily ingredients are desirable for the skin moisturizing and protective properties. Suitable oils include silicones, esters, vegetable oils, synthetic oils, including but not limited to those set forth herein. The oils may be volatile or nonvolatile, and are preferably in the form of a pourable liquid at room temperature. The term "volatile" means that the oil has a measurable vapor pressure, or a vapor pressure of at least about 2 mm. of mercury [267 Pa] at 20° C. The term "nonvolatile" means that the oil has a vapor pressure of less than about 2 mm. of mercury [267 Pa] at 20° C.

### 1. Volatile Oils

Suitable volatile oils generally have a viscosity ranging from about 0.5 to 5 centistokes 25° C. and include linear silicones, cyclic silicones, paraffinic hydrocarbons, or mixtures thereof.

### (a). Volatile Silicones

Cyclic silicones are one type of volatile silicone that may be used in the composition. Such silicones have the general formula: 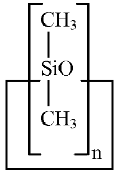 where n=3-6, preferably 4, 5, or 6.

Also suitable are linear volatile silicones, for example, those having the general formula:

(CH₃)₃Si-O-[Si(CH₃)₂-O]ₙ-Si(CH₃)₃

where n=0, 1, 2, 3, 4, or 5, preferably 0, 1, 2, 3, or 4.

Cyclic and linear volatile silicones are available from various commercial sources including Dow Corning Corporation and General Electric. The Dow Corning linear volatile silicones are sold under the tradenames Dow Corning 244, 245, 344, and 200 fluids. These fluids include hexamethyldisiloxane (viscosity 0.65 centistokes (abbreviated cst)), octamethyltrisiloxane (1.0 cst), decamethyltetrasiloxane (1.5 cst), dodecamethylpentasiloxane (2 cst) and mixtures thereof, with all viscosity measurements being at 25° C.

Suitable branched volatile silicones include alkyl trimethicones such as methyl trimethicone, a branched volatile silicone having the general formula: 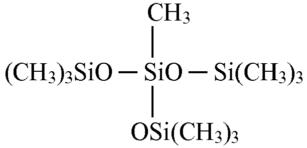 Methyl trimethicone may be purchased from Shin-Etsu Silicones under the tradename TMF-1.5, having a viscosity of 1.5 centistokes at 25° C.

### (b). Volatile Paraffinic Hydrocarbons

Also suitable as the volatile oils are various straight or branched chain paraffinic hydrocarbons having 5, 6, 7, 8, 9, 10, 11, 12, 13, 14, 15, 16, 17, 18, 19, or 20 carbon atoms, more preferably 8 to 16 carbon atoms. Suitable hydrocarbons include pentane, hexane, heptane, decane, dodecane, tetradecane, tridecane, and C₈₋₂₀ isoparaffins as disclosed in U.S. Pat. Nos. 3,439,088 and 3,818,105.
Preferred volatile paraffinic hydrocarbons have a molecular weight of 70-225, preferably 160 to 190 and a boiling point range of 30 to 320, preferably 60 to 260° C., and a viscosity of less than about 10 cst. at 25° C. Such paraffinic hydrocarbons are available from EXXON under the ISOPARS trademark, and from the Permethyl Corporation. Suitable C₁₂ isoparaffins are manufactured by Permethyl Corporation under the tradename Permethyl 99A. Various C₁₆ isoparaffins commercially available, such as isohexadecane (having the tradename Permethyl R), are also suitable.

### 2. Non-Volatile Oils

A variety of nonvolatile oils are also suitable for use in the compositions of the invention. The nonvolatile oils generally have a viscosity of greater than about 5 to 10 centistokes at 25° C., and may range in viscosity up to about 1,000,000 centipoise at 25° C. Examples of nonvolatile oils include, but are not limited to:

### (a). Esters

Suitable esters are mono-, di-, and triesters. The composition may comprise one or more esters selected from the group, or mixtures thereof.

### (i) Monoesters

Monoesters are defined as esters formed by the reaction of a monocarboxylic acid having the formula R-COOH, wherein R is a straight or branched chain saturated or unsaturated alkyl having 2 to 45 carbon atoms, or phenyl; and an alcohol having the formula R-OH wherein R is a straight or branched chain saturated or unsaturated alkyl having 2-30 carbon atoms, or phenyl. Both the alcohol and the acid may be substituted with one or more hydroxyl groups. Either one or both of the acid or alcohol may be a "fatty" acid or alcohol, and may have from about 6 to 30 carbon atoms, more preferably 12, 14, 16, 18, or 22 carbon atoms in straight or branched chain, saturated or unsaturated form. Examples of monoester oils that may be used in the compositions of the invention include hexyl laurate, butyl isostearate, hexadecyl isostearate, cetyl palmitate, isostearyl neopentanoate, stearyl heptanoate, isostearyl isononanoate, steary lactate, stearyl octanoate, stearyl stearate, isononyl isononanoate, and so on.

### (ii). Diesters

Suitable diesters are the reaction product of a dicarboxylic acid and an aliphatic or aromatic alcohol or an aliphatic or aromatic alcohol having at least two substituted hydroxyl groups and a monocarboxylic acid. The dicarboxylic acid may contain from 2 to 30 carbon atoms, and may be in the straight or branched chain, saturated or unsaturated form. The dicarboxylic acid may be substituted with one or more hydroxyl groups. The aliphatic or aromatic alcohol may also contain 2 to 30 carbon atoms, and may be in the straight or branched chain, saturated, or unsaturated form. Preferably, one or more of the acid or alcohol is a fatty acid or alcohol, i.e. contains 12-22 carbon atoms. The dicarboxylic acid may also be an alpha hydroxy acid. The ester may be in the dimer or trimer form. Examples of diester oils that may be used in the compositions of the invention include diisotearyl malate, neopentyl glycol dioctanoate, dibutyl sebacate, dicetearyl dimer dilinoleate, dicetyl adipate, diisocetyl adipate, diisononyl adipate, diisostearyl dimer dilinoleate, diisostearyl fumarate, diisostearyl malate, dioctyl malate, and so on.

### (iii). Triesters

Suitable triesters comprise the reaction product of a tricarboxylic acid and an aliphatic or aromatic alcohol or alternatively the reaction product of an aliphatic or aromatic alcohol having three or more substituted hydroxyl groups with a monocarboxylic acid. As with the mono- and diesters mentioned above, the acid and alcohol contain 2 to 30 carbon atoms, and may be saturated or unsaturated, straight or branched chain, and may be substituted with one or more hydroxyl groups. Preferably, one or more of the acid or alcohol is a fatty acid or alcohol containing 12 to 22 carbon atoms. Examples of triesters include esters of arachidonic, citric, or behenic acids, such as triarachidin, tributyl citrate, triisostearyl citrate, tri C₁₂-₁₃ alkyl citrate, tricaprylin, tricaprylyl citrate, tridecyl behenate, trioctyldodecyl citrate, tridecyl behenate; or tridecyl cocoate, tridecyl isononanoate, and so on.

Esters suitable for use in the composition are further described in the C.T.F.A. Cosmetic Ingredient Dictionary and Handbook, Eleventh Edition, 2006, under the classification of "Esters".

### (b). Hydrocarbon Oils

It may be desirable to incorporate one or more nonvolatile hydrocarbon oils into the composition used in the method of the invention. Suitable nonvolatile hydrocarbon oils include paraffinic hydrocarbons and olefins, preferably those having greater than about 20 carbon atoms. Examples of such hydrocarbon oils include C₂₄₋₂₈ olefins, C₃₀₋₄₅ olefins, C₂₀₋₄₀ isoparaffins, hydrogenated polyisobutene, polyisobutene, polydecene, hydrogenated polydecene, mineral oil, pentahydrosqualene, squalene, squalane, and mixtures thereof. In one preferred embodiment such hydrocarbons have a molecular weight ranging from about 300 to 1000 Daltons.

### (c). Glyceryl Esters of Fatty Acids

Synthetic or naturally occurring glyceryl esters of fatty acids, or triglycerides, are also suitable for use in the compositions. Both vegetable and animal sources may be used. Examples of such oils include castor oil, lanolin oil, C₁₀₋₁₈ triglycerides, caprylic/capric/triglycerides, sweet almond oil, apricot kernel oil, sesame oil, camelina sativa oil, tamanu seed oil, coconut oil, corn oil, cottonseed oil, linseed oil, ink oil, olive oil, palm oil, illipe butter, rapeseed oil, soybean oil, grapeseed oil, sunflower seed oil, walnut oil, and the like.

Also suitable are synthetic or semi-synthetic glyceryl esters, such as fatty acid mono-, di-, and triglycerides which are natural fats or oils that have been modified, for example, mono-, di- or triesters of polyols such as glycerin. In an example, a fatty (C₁₂₋₂₂) carboxylic acid is reacted with one or more repeating glyceryl groups. glyceryl stearate, diglyceryl diiosostearate, polyglyceryl-3 isostearate, polyglyceryl-4 isostearate, polyglyceryl-6 ricinoleate, glyceryl dioleate, glyceryl diisotearate, glyceryl tetraisostearate, glyceryl trioctanoate, diglyceryl distearate, glyceryl linoleate, glyceryl myristate, glyceryl isostearate, PEG castor oils, PEG glyceryl oleates, PEG glyceryl stearates, PEG glyceryl tallowates, and so on.

### (d). Nonvolatile Silicones

Nonvolatile silicone oils, both water soluble and water insoluble, are also suitable for use in the composition. Such silicones preferably have a viscosity ranging from about greater than 5 to 800,000 cst, preferably 20 to 200,000 cst at 25° C. Suitable water insoluble silicones include amine functional silicones such as amodimethicone.

For example, such nonvolatile silicones may have the following general formula: 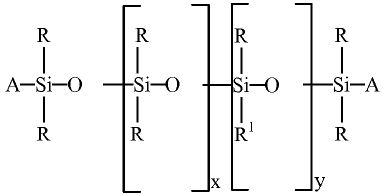 wherein R and R' are each independently C₁₋₃₀ straight or branched chain, saturated or unsaturated alkyl, phenyl or aryl, trialkylsiloxy, and x and y are each independently 1-1,000,000; with the proviso that there is at least one of either x or y, and A is alkyl siloxy endcap unit. Preferred is where A is a methyl siloxy endcap unit; in particular trimethylsiloxy, and R and R' are each independently a C₁₋₃₀ straight or branched chain alkyl, phenyl, or trimethylsiloxy, more preferably a C₁₋₂₂ alkyl, phenyl, or trimethylsiloxy, most preferably methyl, phenyl, or trimethylsiloxy, and resulting silicone is dimethicone, phenyl dimethicone, diphenyl dimethicone, phenyl trimethicone, or trimethylsiloxyphenyl dimethicone. Other examples include alkyl dimethicones such as cetyl dimethicone, and the like wherein at least one R is a fatty alkyl (C₁₂, C₁₄, C₁₆, C₁₈, C₂₀, or C₂₂), and the other R is methyl, and A is a trimethylsiloxy endcap unit, provided such alkyl dimethicone is a pourable liquid at room temperature. Phenyl trimethicone can be purchased from Dow Corning Corporation under the tradename 556 Fluid. Trimethylsiloxyphenyl dimethicone can be purchased from Wacker-Chemie under the tradename PDM-1000. Cetyl dimethicone, also referred to as a liquid silicone wax, may be purchased from Dow Corning as Fluid 2502, or from DeGussa Care & Surface Specialties under the trade names Abil Wax 9801, or 9814.

### F. Vitamins and Antioxidants

It may be desirable to incorporate one or more vitamins or antioxidants in the composition used in the method of the invention. If present, suggested ranges are from about 0.001 to 20%, preferably from about 0.005 to 15%, more preferably from about 0.010 to 10%. Preferably such vitamins, vitamin derivatives and/or antioxidants are operable to scavenge free radicals in the form of singlet oxygen. Such vitamins may include tocopherol or its derivatives such as tocopherol acetate, tocopherol ferulate; ascorbic acid or its derivatives such as ascorbyl palmitate, magnesium ascorbyl phosphate; Vitamin A or its derivatives such as retinyl palmitate; or vitamins D, K, B, or derivatives thereof.

### G. Preferred Compositions

Preferred compositions used in the method of the invention are in the aqueous solution or emulsion form.

More preferred is where the composition used in the method of the invention comprises at least one nonionic organic surfactant which is an alkoxylated alcohol and the at least one oil is an organic ester or hydrocarbon.

The invention will be further described in connection with the following examples which are set forth for the purposes of illustration only.

### EXAMPLE 1

A skin treatment composition is prepared as follows:

| Ingredient | w/w% |
|---|---|
| Oleth-3 phosphate | 0.45 |
| Oleth-3 | 0.35 |
| Oleth-5 | 0.24 |
| Butylene glycol | 0.20 |
| Squalane | 0.50 |
| BHT | 0.10 |
| Ethylhexyl methoxycinnamate | 0.10 |
| Choleth-24/ceteth-24 | 0.10 |
| Triethanolamine | 0.11 |
| Retinyl palmitate/zea mays (corn) oil/BHT/BHA | 0.10 |
| Butylene glycol | 1.1 |
| Chamomile | 0.03 |
| Bisabolol | 0.10 |
| Water | QS |
| Methyl paraben | 0.46 |
| PEG-75 | 4.00 |
| Bis-PEG-18 methyl ether dimethyl silane | 2.00 |
| Glycereth-26 | 1.00 |
| Methyl gluceth-20 | 4.00 |
| Trisodium EDTA | 0.10 |
| Pantethine | 0.14 |
| Caffeine | 0.05 |
| Xanthan gum | 0.075 |
| Carbomer | 0.26 |
| Triethanolamine | 0.50 |
| Phenoxyethanol | 0.70 |
| Benzyl alcohol | 0.10 |
| Bifida ferment lysate | 9.40 |
| Water/bifida ferment lysate/hydrogenated lecithin | 3.00 |
| Butylene glycol/water/*Cola Acuminata* extract | 3.00 |
| Sodium ribonucleic acid | 0.01 |
| Cichoric acid | 0.20 |
| Lactobacillus ferment/lecithin/water | 0.05 |
| Water/*Arabidopsis Thaliana* extract/lecithin | 0.05 |
| Phenoxyethanol | 0.02 |
| Sodium hyaluronate | 0.01 |
| FD&C Red No. 4 (1% aqueous solution with butylene glycol) | 0.04 |
| FD&C Yellow No. 5 (1% aqueous solution with butylene glycol) | 0.09 |
| D&C Green No. 5 (0.1% solution with butylene glycol) | 0.001 |

The composition is prepared by combining the ingredients and mixing well to form a liquid. The composition is stored in glass bottles.

### EXAMPLE 2 (Reference)

Cichoric acid and *Ecchinacea purpurea* extract (Symfinity 1298, Symrise) were tested to determine per1 gene expression.

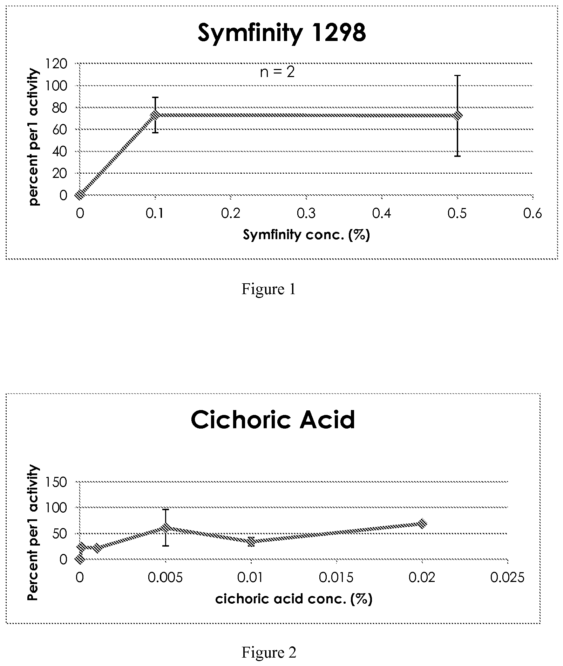

Normal human epidermal keratinocytes (NHEK) were diluted in EpiLife Media MEP1500CA with supplement S001-5 added (Gibco, Cascade Biologics, Invitrogen) to form a concentration of 3 x 10⁴ and plated onto a black walled 96 well microtiter plate by adding 100 µl in each well. The plate was incubated for 3 hours at 37° C. in 5% CO₂. The media was removed and remaining adhered cells rinsed with EpiLife Media MEP1500CA, supplement free. A plasmid solution was prepared by diluting Tris-EDTA pH 8.0 buffer with plasmid pGL4.11 (DNA 2.0, Carlsbad, CA) to form a 1 mg/ml solution. This plasmid contained a perl gene sequence and a luciferase reporter gene sequence. A transfection mixture was prepared by diluting EpiLife Media MEP1500CA, supplement free, to form a solution with 0.31 µg/ml of plasmid (obtained by adding appropriate amount of plasmid solution prepared above), 1.28% Plus™ Reagent (Catalog No. 11514-015, Invitrogen, Carlsbad, CA), and 3.22% Lipofectamine™ Transfection Reagent (Catalog No. 18324-012, Invitrogen, Carlsbad, CA). Transfection mixture, 40 µl, was added to each well and the plate incubated for 4 hours at 37° C. in 5% CO₂. Then 80 µl of EpiLife Media MEP1500CA with supplement S001-5 was added to the well containing the control (media alone), the transfection mixture control (transfection mixture without any active ingredient). Test wells and transfection control wells. To the test wells 80 µl of test material solution (test material diluted in EpiLife Media MEP1500CA with supplement S001-5) was added. The wells were incubated for 16 hours at 37° C. in 5% CO₂. Immediately prior to reading, Bright-Glo™ (Pro-Mega, Madison WI) was added to each well plate in the same volume as is currently in the well (e.g. a 1:1 dilution). Luminescence was measured in an LMax™ Microplate Luminometer (Molecular Devices, Sunnyvale, CA). The results are set forth in figures 1 and 2. These results demonstrate that both *Ecchinacea purpurea* extract and cichoric acid stimulate perl gene expression in keratinocytes.

## Claims

1. A cosmetic method for treating adverse effects of the natural aging process of the skin, the method comprising increasing and/or synchronizing perl gene expression in skin cells having decreased, irregular, or asynchronous perl gene expression due to the natural aging process by treating the skin cells with a composition comprising an effective amount of cichoric acid, and a DNA repair enzyme;
wherein the cichoric acid is in the form of an extract of *Echinacea purpurea*;
wherein the DNA repair enzyme is in the form of *Bifida* Ferment Lysate; and
wherein the composition is in the form of a skin cream, lotion, foundation makeup, lipstick, concealer, blush, serum, eye shadow, cleanser or toner.

2. The method of claim 1 wherein the effective amount of cichoric acid ranges from about 0.00001 to 10% by weight of the total composition that is applied to the skin cells.

3. The method of claim 1 wherein the skin cells are keratinocytes.

4. The method of claim 1 where the composition is applied to the skin in the evening prior to retiring.

5. A composition for use in a method for increasing and/or synchronizing perl gene expression in skin cells having decreased, irregular, or asynchronous perl gene expression due to exposure to UV radiation comprising treating the skin cells with the composition;
wherein the composition comprises an effective amount of cichoric acid, and a DNA repair enzyme;
wherein the cichoric acid is in the form of an extract of *Echinacea purpurea*;
wherein the DNA repair enzyme is in the form of *Bifida* Ferment Lysate; and
wherein the composition is in the form of a skin cream, lotion, foundation makeup, lipstick, concealer, blush, serum, eye shadow, cleanser or toner.

## Patentansprüche

1. Kosmetisches Verfahren zum Behandeln ungünstiger Wirkungen des natürlichen Alterungsprozesses der Haut, wobei das Verfahren Folgendes umfasst: Erhöhen und/oder Synchronisieren der perl-Genexpression in Hautzellen mit erniedrigter, irregulärer oder asynchroner perl-Genexpression aufgrund des natürlichen Alterungsprozesses durch Behandeln der Hautzellen mit einer Zusammensetzung, die eine wirksame Menge Chichoriensäure und ein DNA-Reparaturenzym umfasst;
wobei die Chichoriensäure in Form eines Extrakts von *Echinacea purpurea* vorliegt;
wobei das DNA-Reparaturenzym in Form von Bifida-Fermentlysat vorliegt; und
wobei die Zusammensetzung in Form einer/eines Hautcreme, Lotion, Foundation-Makeups, Lippenstifts, Concealers, Rouges, Serums, Lidschattens, Reinigers oder Gesichtswassers vorliegt.

2. Verfahren nach Anspruch 1, wobei die wirksame Menge Chichoriensäure von etwa 0,00001 bis 10 Gew.-% der Gesamtzusammensetzung, die auf die Hautzellen aufgetragen wird, reicht.

3. Verfahren nach Anspruch 1, wobei die Hautzellen Keratinozyten sind.

4. Verfahren nach Anspruch 1, wobei die Zusammensetzung am Abend vor dem Zubettgehen auf die Haut aufgetragen wird.

5. Zusammensetzung zur Verwendung bei einem Verfahren zum Erhöhen und/oder Synchronisieren der perl-Genexpression in Hautzellen mit erniedrigter, irregulärer oder asynchroner perl-Genexpression aufgrund der Exposition gegenüber UV-Strahlung, umfassend das Behandeln der Hautzellen mit der Zusammensetzung, wobei die Zusammensetzung eine wirksame Menge Chichoriensäure und ein DNA-Reparaturenzym umfasst;
wobei die Chichoriensäure in Form eines Extrakts von *Echinacea purpurea* vorliegt;
wobei das DNA-Reparaturenzym in Form von *Bifida*-Fermentlysat vorliegt; und
wobei die Zusammensetzung in Form einer/eines Hautcreme, Lotion, Foundation-Makeups, Lippenstifts, Concealers, Rouges, Serums, Lidschattens, Reinigers oder Gesichtswassers vorliegt.

## Revendications

1. Procédé esthétique destiné au traitement des effets indésirables du processus de vieillissement naturel de la peau, le procédé comprenant l'augmentation et/ou la synchronisation de l'expression du gène per1 dans des cellules cutanées présentant une expression du gène per1 réduite, irrégulière ou non synchronisée due au processus de vieillissement naturel en traitant les cellules cutanées avec une composition comprenant une quantité efficace d'acide cichorique, et une enzyme réparatrice d'ADN ;
dans lequel l'acide cichorique est sous la forme d'un extrait d'*Echinacea purpurea* ;
dans lequel l'enzyme réparatrice d'ADN est sous la forme de *Bifida* Ferment Lysate ;
dans lequel la composition est sous la forme d'une crème pour la peau, d'une lotion, d'un fond de teint, d'un rouge à lèvres, d'un correcteur, d'un fard à joues, d'un sérum, d'un fard à paupières, d'un démaquillant ou d'un tonique.

2. Procédé selon la revendication 1 dans lequel la quantité efficace d'acide cichorique est comprise entre environ 0,00001 et 10 % en poids de la composition totale qui est appliquée sur les cellules cutanées.

3. Procédé selon la revendication 1 dans lequel les cellules cutanées sont des kératinocytes.

4. Procédé selon la revendication 1 dans lequel la composition est appliquée à la peau le soir avant le coucher.

5. Composition à utiliser dans un procédé destiné à augmenter et/ou à synchroniser l'expression du gène per1 dans des cellules cutanées présentant une expression du gène per1 réduite, irrégulière ou non synchronisée en raison d'une exposition aux rayonnements UV comprenant le traitement des cellules cutanées avec la composition ;
dans laquelle la composition comprend une quantité effective d'acide cichorique, et une enzyme réparatrice d'ADN ;
dans laquelle l'acide cichorique est sous la forme d'un extrait d'*Echinacea purpurea* ;
dans laquelle l'enzyme réparatrice d'ADN est sous la forme de *Bifida* Ferment Lysate ;
dans laquelle la composition est sous la forme d'une crème pour la peau, d'une lotion, d'un fond de teint, d'un rouge à lèvres, d'un correcteur, d'un fard à joues, d'un sérum, d'un fard à paupières, d'un démaquillant ou d'un tonique.
